Europäisches Patentamt

European Patent Office

Office européen des brevets

(11) Numéro de publication : **0 449 924 B1**

(19)

(12)

# FASCICULE DE BREVET EUROPEEN

(45) Date de publication du fascicule du brevet :
28.09.94 Bulletin 94/39

(51) Int. Cl.⁵ : **G01N 33/24, C12Q 1/42**

(21) Numéro de dépôt : **90900902.9**

(22) Date de dépôt : **22.12.89**

(86) Numéro de dépôt international :
**PCT/FR89/00674**

(87) Numéro de publication internationale :
**WO 90/07112 28.06.90 Gazette 90/15**

(54) PROCEDE DE DETERMINATION DE L'ACTIVITE BIOLOGIQUE D'UN SOL.

(30) Priorité : **22.12.88 FR 8817008**

(43) Date de publication de la demande :
**09.10.91 Bulletin 91/41**

(45) Mention de la délivrance du brevet :
**28.09.94 Bulletin 94/39**

(84) Etats contractants désignés :
**BE CH DE ES GB IT LI NL**

(56) Documents cités :
**EP-A- 0 085 348
CHEMICAL ABSTRACTS, vol. 94, no. 5, 02
fevrier 1981, Columbus, OH (US); P. NANNI-
PIERI et al., p. 451, no. 29252k
BIOLOGICAL ABSTRACTS, vol. 87, no. 7, 1989,
Philadelphia, PA (US); T. SUTTNER et al., p.
AB-1102, no. 77236**

(56) Documents cités :
**BIOLOGICAL ABSTRACTS, vol. 78, no. 9, 1989,
Philadelphia, PA (US); W.A. DICK, p.
7257-7258, no. 64394
SOIL SCI. SOC. AM. JOURNAL, vol. 44, 1980; P.
NANNIPIERI et al., pp. 1011-1016
ZENTRALBL. MIKROBIOL., vol. 143, 1988; T.
SUTTNER et al., pp. 569-573 ;**

(73) Titulaire : **BOURGUIGNON, Claude
Marey-sur-Tille
F-21120 Is-sur-Tille (FR)**

(72) Inventeur : **BOURGUIGNON, Claude
Marey-sur-Tille
F-21120 Is-sur-Tille (FR)**

(74) Mandataire : **Bruder, Michel et al
Cabinet Michel Bruder
Conseil en Brevets
10, rue de la Pépinière
F-75008 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne, d'une manière générale, un procédé de détermination de l'activité biologique d'un sol.

A l'heure actuelle, lorsque l'on désire estimer l'activité biologique d'un sol, on doit mettre en oeuvre des méthodes longues et complexes, telles que les méthodes biocidales (Jenkinsson et Ladd, 1981) ou les méthodes de mesure du dégagement de gaz carbonique ou de la teneur en adénosine tri-phosphate (Maire, 1987). Il s'ensuit que l'on se heurte à un coût d'analyse trop élevé ou à une durée excessive des opérations pour pouvoir appliquer ces méthodes à des déterminations de routine.

L'article de Suttner et al : "correlation between the arginine ammonification, enzyme activities, microbial biomass, physical and chemical properties of different soils" Zentralbl. 569-573 1988, décrit un procédé qui se réfère à des corrélations entre l'ammonification de l'arginine et les activités de la catalase, de la déshydrogénase, de la protéase, de la phosphtase alcaline et de la saccharase c'est-à-dire un certain nombre d'enzymes. Des relations sont également établies entre l'ammonification de l'arginine, le carbone organique, la teneur totale en azote, la capacité d'échange cationique et la masse volumique moyenne. On peut résumer en disant que cet article effectue la corrélation entre l'ammonification de l'arginine, les activités enzymatiques et la teneur en carbone des sols, et leurs capacités d'échange cationique. Il n'y a donc aucune corrélation entre l'activité de la phosphatase alcaline et la teneur en carbone, ainsi qu'entre laphosphatase alcaline et la capacité d'échange cationique et la surface interne du sol.

Or, il serait souhaitable de pouvoir disposer d'un procédé qui permette de mesurer l'activité biologique ou microbienne d'un sol, qui soit suffisamment simple, rapide et économique pour que les agriculteurs puissent y avoir recours à titre ordinaire.

Ce but est atteint selon l'invention en ce sens qu'elle propose un procédé qui consiste :

- à établir une corrélation entre, d'une part, la teneur en phosphatase alcaline d'une série de sols de référence dont l'activité biologique est jugée satisfaisante et, d'autre part, d'autres caractéristiques physiques et/ou chimiques de cette série de sols de référence,
- à mesurer la teneur en phosphatase alcaline et lesdites autres caractéristiques du sol objet de la détermination, et
- à comparer les résultats desdites mesures à la corrélation établie à partir des sols de référence.

La teneur en phosphatase alcaline peut être déterminée indirectement par la mesure de son activité enzymatique, laquelle peut être mise en évidence, par exemple, par la dégradation du phosphate de p-nitrophényle en p-nitrophénol.

Le choix s'est porté sur la phosphatase alcaline plutôt que sur une autre enzyme car cette enzyme répond à quatre critères importants :

- elle intervient dans une voie métabolique fondamentale (cycle de l'ATP) et, donc, elle existe chez tous les microorganismes ;
- elle n'existe pas dans les racines des plantes,
- son activité ne dépend pas de la teneur en phosphore des sols (la phosphatase acide est, elle, inhibée par les fortes teneurs en phosphore), et
- sa technique de dosage est simple et peu coûteuse.

Par suite, le dosage de l'activité de cette enzyme dans un échantillon de sol reflète bien, et de manière économique, l'activité biologique microbienne du sol en question.

Il est toutefois nécessaire de corréler cette mesure avec une autre caractéristique du sol.

Dans un premier mode de mise en oeuvre de l'invention, une autre dite caractéristique est la surface interne du sol, c'est-à-dire la surface déployée par un quantité donnée de sol étalée en couche élémentaire.

Dans un second mode de mise en oeuvre de l'invention, une autre dite caractéristique avec laquelle est corrélée la mesure de la phosphatase alcaline est la teneur en carbone organique du sol.

Dans un troisième mode de mise en oeuvre de l'invention, une autre dite caractéristique avec laquelle est corrélée la mesure de la phosphatase alcaline est la capacité d'échange cationique du sol.

Les mesures faites sur les sols de référence permettent de tirer de la représentation graphique de leur corrélation une droite de régression et l'ensemble axes/droite de régression forme un abaque que l'on utilise ensuite pour estimer l'activité biologique du sol soumis à la détermination.

Plus précisément, on a déterminé expérimentalement, dans le cadre du premier mode de mise en oeuvre évoqué plus haut, que la droite de régression obtenue à partir des couples de mesures

. activité enzymatique de la phosphatase alcaline exprimée en µg de p-nitrophénol produit à partir de phosphate de p-nitrophényle /g de sol sec/heure, portée en ordonnée

. surface interne du sol exprimée en $m^2$/g de sol, portée en abscisse,

fait apparaître un facteur de régression r de l'ordre de $\varnothing$,95.

Dans le cadre du second mode de mise en oeuvre de l'invention, la droite de régression obtenue à partir des couples de mesures

. activité enzymatique de la phosphatase alcaline exprimée en µg de p-nitrophénol produit à partir de phosphate de p-nitrophényle /g de sol sec/heure, portée en ordonnée,

. teneur en carbone organique du sol exprimé en ‰, portée en abscisse,

fait apparaître un facteur de régression r de l'ordre de Ø,75.

Dans le cadre du troisième mode de mise en oeuvre de l'invention, la droite de régression obtenue à partir des couples de mesures

. activité enzymatique de la phosphatase alcaline exprimée en µg de p-nitrophénol produit à partir de phosphate de p-nitrophényle /g de sol sec/heure, portée en ordonnée,

. capacité d'échange cationique du sol exprimée en milliéquivalents/g de sol sec portée en abscisse,

fait apparaître un facteur de régression r de l'ordre de Ø,65.

Quel que soit le mode de mise en oeuvre choisi, on compare les valeurs de l'activité enzymatique de la phosphatase alcaline données, d'une part, par la courbe de régression et, d'autre part, par la mesure effectuée sur le sol soumis à la détermination, pour la même valeur d'autres caractéristiques choisies - surface interne du sol, teneur en carbone organique du sol ou capacité d'échange cationique du sol - et l'on interprète le résultat à partir des hyperboles de confiance comme suit :

- valeur dans les hyperboles à 95 % : bon
- valeur dans les hyperboles à 90 % : insuffisant ou fort
- valeur hors des hyperboles à 9Ø% : mauvais ou excessif

Il a été vérifié sur plus de deux cents sols agricoles des zones tempérées et tropicales que le procédé selon l'invention donne des résultats parfaitement corrélés avec ceux des méthodes complexes évoquées plus haut.

Le protocole opératoire des trois variantes du procédé selon l'invention est décrit ci-après, étant toutefois entendu que les techniques de dosage sont en elles-mêmes connues et que l'invention réside dans la corrélation des mesures.

Pour les sols de référence, dans ces exemples, on a utilisé des prélèvements sur des parcelles d'essais de longue durée (Déhérain, Versailles, Dijon, ITCF) dont on connaît bien les fonctionnements et les rendements des cultures.

## EXEMPLE 1

Corrélation entre la mesure de l'activité enzymatique de la phosphatase alcaline et celle la surface interne du sol.

### 1) Mesure de l'activité enzymatique de la phosphatase acide

On utilise la méthode de TABATABAI et BRENNER, 1982, In. Methods of Soil Analysis, Part 2 by A.L. PAGE et al. American Society of Agronomy, modifiée par Claude BOURGUIGNON, 1987.

Le sol est tamisé à 2mm. Une partie est exposée pendant 3 jours à l'air libre et une autre partie est utilisée immédiatement. Dans les deux cas (sol frais et sol séché à l'air), le protocole est le même :

### a - Mesure de l'humidité

On pèse un échantillon de 5Ø g de sol séché à l'air ou frais, on le met à sécher pendant 24 heures dans une étuve à 1Ø5°C et on le pèse de nouveau après refroidissement dans un dessicateur. La différence donne la teneur en humidité exprimée en grammes, teneur dont il faudra tenir compte pour l'expression des résultats (point c4. ci-dessous).

### b - Incubation enzymatique

On introduit une prise d'essai de 1 g de sol (frais ou séché à l'air) dans une fiole Erlenmeyer de 5Ø ml et on ajoute 0,2 ml de toluène, 4 ml d'une solution tampon à pH 1Ø,8 (Diluer dans 8ØØ ml d'eau, 2ØØ ml d'une solution mère contenant 12,1 g de tampon Tris (hydroxyméthylaminométhane), 11,6 g d'acide malique, 14 g d'acide citrique et 6,3 g d'acide borique dans 488 ml de NaOH, 1N. Diluer avec $H_2O$ et ramener à 1 litre) et 1 ml d'une solution de phosphate de p-nitrophényle. On agite, on bouche la fiole et on incube dans un agitateur fonctionnant à 5O révolutions/mn (5Ø rpm) à 37°C pendant 1 heure. Sous l'action de la phosphatase acide contenue dans la prise d'essai, une quantité plus ou moins grande de phosphate de p-nitrophényle est convertie en p-nitrophénol.

### c - Mesure spectrophotométrique

Elle consiste à mesurer la quantité de p-nitrophénol produite, laquelle est fonction de l'activité de la phosphatase alcaline.

### c1.Témoin :

On met 1g de sol à incuber comme précédemment, mais sans adjonction de phosphate de p-nitrophényle. Après incubation, on ajoute 1 ml d'une solution aqueuse de $CaCl_2.H_2O$, à la concentration de 73,5g/litre et 4 ml d'une solution aqueuse de NaOH Ø,5 Molaire. On agite, puis on filtre sur papier Whatman n° 4Ø et lit au spectrophotomètre sous 41Øµm.

### c2.Courbe d'étalonnage (solution standard colorée) :

On dilue 1 ml d'une solution standard de p-nitrophénol (1g/l) dans 1ØØ ml d'eau et l'on distribue la dilution résultante dans des fioles Erlenmeyer, à raison respectivement de O, 1, 2, 3, 4 et 5 ml. On ajuste à 5 ml avec de l'eau, ce qui donne une gamme de concentrations allant de Ø à 5Ø µg de p-nitrophénol. On mesure l'absorption de cette gamme à 41Øµm et on trace la courbe correspondante.

c3. prise d'essai

A l'issue de la période d'incubation enzymatique, on ajoute, comme pour le témoin, 1 ml d'une solution aqueuse de CaCl₂.H₂O, à la concentration de 73,5g/litre et 4 ml d'une solution aqueuse de NaOH Ø,5 Molaire. On agite, puis on filtre sur papier Whatman n° 4Ø. Le spectrophomètre étant calé en fonction de l'erreur introduite par le témoin, on mesure l'absorption à 41Øµm.

c4. expression des résultats

A partir de cette absorption et de la courbe d'étalonnage obtenue sous c2, on détermine la concentration en p-nitrophénol de la prise d'essai et donc, indirectement, l'activité enzymatique de la phosphatase alcaline.

Cette activité est exprimée en µg de p-nitrophénol produit en une heure par lg de sol sec, c'est-à-dire en tenant compte de la mesure de l'humidité effectuée sous a ci-dessus.

2 - Mesure de la surface interne du sol

La mesure utilisée est celle de la technique dite "au bleu de méthylène" du Laboratoire Central des Ponts et Chaussées. Le dosage consiste à faire des ajouts de bleu de méthylène (1Ø g/l) dans une suspension contenant la prise d'essai jusqu'au recouvrement des particules du sol par une couche monomoléculaire de ce colorant.

a - Préparation de l'échantillon de sol

Un prélèvement de sol est tamisé à 2mm et une prise d'essai de 1 g est prélevée et mise dans 1ØØ ml d'eau permutée. On y ajoute 1Ø ml d'une solution aqueuse d'hexamétaphosphate de sodium à 1ØØ g/l. On met ensuite au mixeur pendant 3Ø secondes.

b - Fixation du bleu de méthylène

On fait des ajouts de 2 ml de solution de bleu de méthylène (1Øg/l) jusqu'à ce qu'une goutte du mélange suspension de sol/bleu de méthylène, déposée sur du papier filtre, laisse apparaître une auréole bleu clair autour de la tache résultante.

c - Expression des résultats

La surface interne est exprimée en m²/g de sol de la façon suivante :

Surface interne = (x.s) (1/y)

formule dans laquelle :

x = volume en ml de solution de bleu de méthylène nécessaire pour recouvrir 1g de sol

s = surface en m² déployée par lg de bleu de méthylène, en l'occurence 19,85 m²

y = pourcentage pondéral d'argile dans le sol.

3 - Corrélation

Une série de mesures a été faite sur des échantillons de sols de référence considérés comme satisfaisants sur le plan de leur activité biologique.

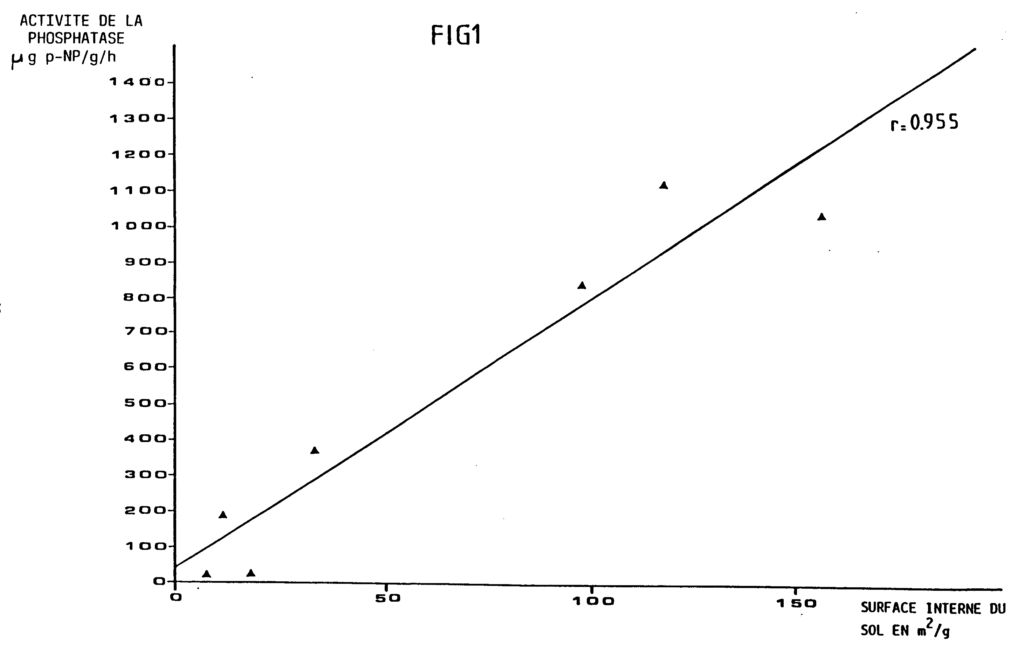

Les résultats sont exprimés graphiquement à la figure 1 du dessin annexé.

A partir de ces résultats, on a pu établir une droite de régression dont le coefficient de régression r est de Ø,955. On obtient ainsi un abaque de corrélation.

4 - Exploitation

En procédant aux mêmes mesures de l'activité enzymatique de la phosphatase alcaline et de la surface interne sur un échantillon de sol dont on veut déterminer l'activité biologique et en comparant les résultats obtenus avec l'abaque issue de l'étape 3, on peut juger de cette activité biologique.

EXEMPLE 2

Corrélation entre la mesure de l'activité enzymatique de la phosphatase alcaline et celle de la teneur en carbone organique.

1) Mesure de l'activité enzymatique de la phosphatase acide

On procède comme à l'exemple 1 et le résultat obtenu est exprimé de la même façon.

2) Mesure de la teneur en carbone organique

La méthode utilisée est celle de ANNE, P. 1945; Ann. Agron. 15:161-172, modifiée par la norme AFNOR d'analyse des sols. Elle consiste à oxyder le carbone organique par une solution sulfo-chromique à l'ébullition et à doser les ions Cr⁺⁺⁺ par spectrocolorimétrie à 625 µm.

a - Mode opératoire

On opère sur une prise d'essai de sol pesant de 2ØØ mg à 5 g selon que le sol est supposé contenir de 2Ø à 1 % de carbone. On introduit cette prise d'essai dans un ballon à col rodé, on ajoute 5Ø ml de mélange sulfo-chromique (3Ø g de CrØ₃ dissous dans 54Ø ml d'eau distillée + 500 ml d'acide sulfurique concentré) et 15 ml d'acide sulfurique concentré.

Après avoir adapté au col rodé du ballon un réfrigérant à reflux, on porte le contenu du ballon à l'ébullition pendant 5 mn. On laisse refroidir pendant 3Ø mn, puis on transvase quantitativement dans une fiole jaugée de 2ØØ ml. On laisse reposer une heure, après quoi on décante le liquide clair de la fiole. Après un nouveau repos, de 12 heures cette fois, on décante dans un pilulier à bouchon hermétique.

b - Mesure spectrocolorimètrique

b1. Gamme étalon

On prépare, à partir d'une solution mère de glucose à 1Ø g/l (donc à 4 g de carbone par litre), une gamme de dilutions titrant respectivement Ø, 1Ø, 2Ø, 6Ø, 1ØØ, 2ØØ, 3ØØ, 4ØØ, 5ØØ et 6ØØ µg de carbone/ml. On mesure l'absorption de cette gamme sous 625 µm et on trace la courbe d'étalonnage correspondante.

b2. Mesure

L'appareil étant calé pour tenir compte de l'absorption d'un témoin préparé selon le principe classique rappelé sous l'exemple 1, on mesure l'absorption de la prise d'essai préparée comme indiqué sous a (suspension de liquide d'incubation), et l'on s'en rapporte à la courbe d'étalonnage pour en tirer la concentration en carbone exprimée en µg de carbone par cm³ de suspension (valeur qui peut directement être rapportée au poids de sol sec).

c - Expression des résultats

La teneur en carbone organique est exprimée comme suit :
Teneur en carbone (%) = (z) (1/5Øp) (1ØØ) (1/1ØØ - h)
    formule dans laquelle :
    z = concentration en carbone en µg/g
    p = poids de la prise d'essai (en g)
    h = humidité de la terre (en g) mesurée comme à l'exemple 1.

3 - Corrélation

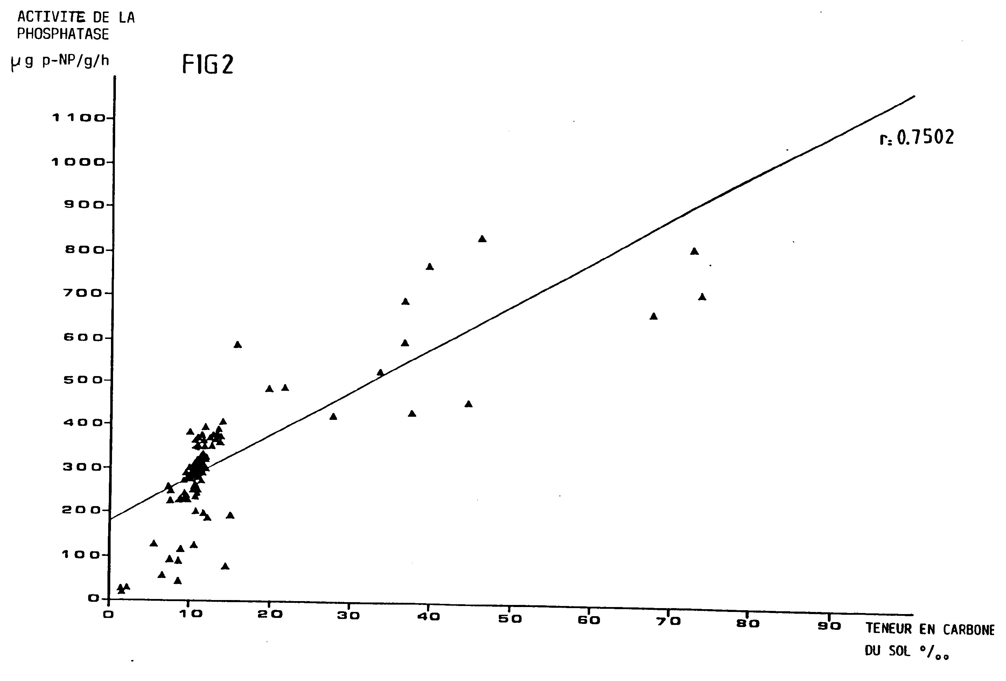

Une série de mesures a été faite sur des échantillons de sols de référence considérés comme satisfaisants sur le plan de leur activité biologique.
Les résultats sont exprimés graphiquement à la figure 2 du dessin annexé.
A partir de ces résultats, on a pu établir une droite de régression dont le coefficient de régression r est de Ø,75Ø2. On obtient ainsi un abaque de corrélation.

4 - Exploitation

En procédant aux mêmes mesures de l'activité enzymatique de la phosphatase alcaline et de la teneur en carbone organique sur un échantillon de sol dont on veut déterminer l'activité biologique et en comparant les résultats obtenus avec l'abaque issu de l'étape 3, on peut juger de cette acticité biologique.

EXEMPLE 3

Corrélation entre la mesure de l'activité enzymatique de la phosphatase alcaline et celle de la capacité d'échange cationique.

1) Mesure de l'activité enzymatique de la phosphatase acide

On procède comme à l'exemple 1 et le résultat obtenu est exprimé de la même façon.

2) Mesure de la capacité d'échange cationique

On utilise la méthode de RHOADES, J.D. 1982. In : Methods of soil analysis, Part 2 by PAGE, A.L. ; MILLER, R.H. and KEENEY, D.R. American Society of Agronomy, Madison, U.S.A.
Cette méthode distingue les sols contenant des carbonates et les sols acides.

2a- Sols contenant des carbonates

2a1 -Mode opératoire

On pèse 4 à 5 g de sol sec que l'on met dans un tube à centrifuger. On ajoute 33 ml d'une solution saturante de NaOAc, Ø,4N et de NaCl Ø,1N avec 6Ø % d'éthanol et ajustée à pH 8,2 avec une solution de NaOH 6N. On agite pendant 5 mn et on centrifuge à 1ØØØ tours/mn pendant 5 mn. On élimine le surnageant et on répète d'opération quatre fois. On ajoute ensuite 33 ml d'une solution de MgNO₃ à Ø,5 N, on agite pendant Smn et on centrifuge jusqu'à éclaircissement du surnageant que l'on verse dans une fiole de 1ØØ ml. On répète l'opération deux fois et on ramène à 1ØØ ml exactement.
On dose, dans des aliquots du contenu de la fiole, le sodium et le chlore présents dans la solution par rapport à des solutions standard (Méthode de RHOADES, section 1Ø-3.4 du même ouvrage).

2a2 - Expression des résultats

La capacité d'échange cationique (C.E.C.), en milliéquivalents pour 1ØØ g de sol sec, est exprimée comme suit :
C.E.C. = (1ØØ/p sol)[(cNa)(1ØØ/v al) - (cCl)(1ØØ/v al)(cNaCl)]

dans laquelle :
- p sol est le poids en grammes de l'échantillon de sol
- cNa est la concentration en Na, exprimée en milliéquivalents par litre, de la solution saturante
- v al est le volume de l'aliquot en ml
- cCl est la concentration en Cl, exprimée en milliéquivalents par litre, de la solution saturante
- cNaCl est la concentration en NaCl, exprimée en milliéquivalents par litre, de la solution saturante

## 2b- Sols acides

### 2b1 - Mode opératoire

On pèse 2 g de sol sec que l'on place dans un tube à centrifugation. On ajoute 2Ø ml d'une solution de $BaCl_2$ Ø,1M. On agite pendant 2 heures et on centrifuge. On jette le surnageant et on répète l'opération deux fois avec 2Ø ml de $BaCl_2$ Ø,ØØ2M. On ajoute 1Ø ml d'une solution de $MgSO_4$ Ø,ØØ5M et on agite pendant 1 heure. On ajuste la capacité d'échange à celle d'une solution de référence de $MgSO_4$ Ø,ØØ15M par ajouts successifs d'une solution de $MgSO_4$ Ø,ØØ5M ou d'eau distillée. On agite doucement pendant une nuit. On pèse le tube et on centrifuge. On détermine le pH et la concentration en Mg du surnageant.

### 2b2 Expression des résultats

La capacité d'échange cationique (C.E.C.), en milliéquivalents pour 1ØØ g de sol sec, est exprimée comme suit :
- si l'on n'a ajouté que de l'eau distillée :
  $$CEC = 1ØØ(Ø,1 - C_1V_3)/p \text{ sol}$$
- si l'on a ajouté du $MgSO_4$
  $$CEC = 1ØØ(Ø,Ø1V_1 - C_1V_3)/p \text{ sol}$$
  formules dans lesquelles :
- p sol = poids en grammes de l'échantillon de sol sec
- $C_1$ = concentration en Mg dans le surnageant en milliéquivalents/ ml
- $V_1$ = volume en ml de la solution de $MgSO_4$ ajoutée
- $V_3$ = volume en ml de la solution finale de surnageant.

## 3 - Corrélation

Une série de mesures a été faite sur des échantillons de sols de référence considérés comme satisfaisants sur le plan de leur activité biologique.

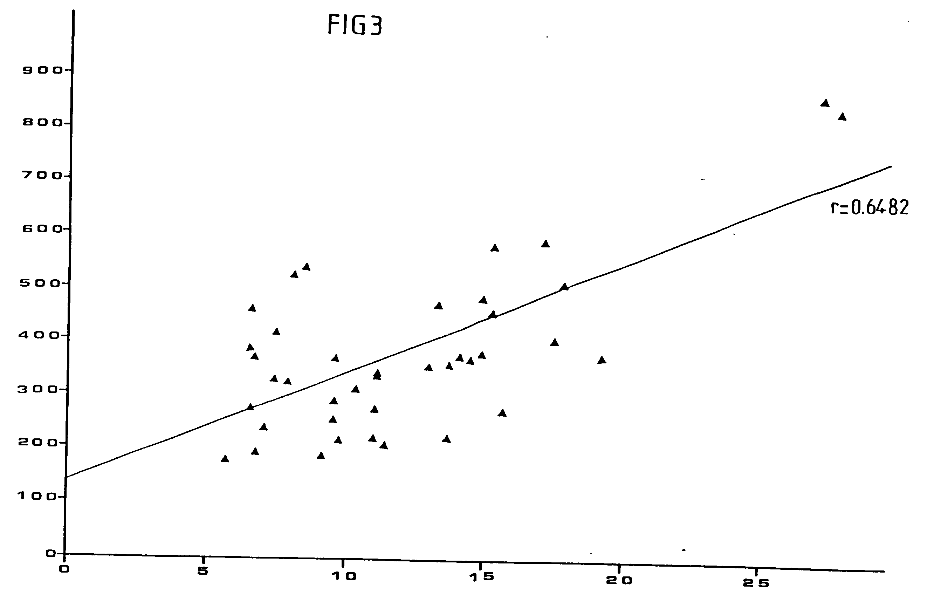

Les résultats sont exprimés graphiquement à la figure 3 du dessin annexé.

A partir de ces résultats, on a pu établir une droite de régression dont le coefficient de régression r est de Ø,6482. On obtient ainsi un abaque de corrélation.

## 4 - Exploitation

En procédant aux mêmes mesures de l'activité enzymatique de la phosphatase alcaline et de la capacité d'échange cationique sur un échantillon de sol dont on veut déterminer l'activité biologique et en comparant les résultats obtenus avec l'abaque issu de l'étape 3, on peut juger de cette acticité biologique.

Que l'on ait recours à l'un ou l'autre mode de mise en oeuvre du procédé selon l'invention, on n'a à effectuer que des opérations simples, rapides et peu coûteuses. On peut, par suite, les appliquer couramment aux sols agricoles en vue d'aboutir à une "analyse conseil". Cela est d'autant plus utile que l'intensification de l'agriculture a conduit à une perte de matière organique dans les sols avec une baisse consécutive de leur activité microbienne. Le procédé selon l'invention permet aux agriculteurs de juger de l'état de leur sol ainsi que de vérifier l'efficacité de tel ou tel produit dans le maintien ou l'augmentation de son activité biologique.

Il est bien entendu que la présente invention n'est pas limitée aux modes opératoires décrits ci-dessus, à titre d'exemples. En particulier, l'activité enzymatique de la phosphatase alcaline pourrait être déterminée en utilisant un autre substrat que le phosphate de p-nitrophényle, ou même la phosphatase alcaline pourrait être dosée directement par tout procédé approprié. De même, la teneur en carbone, la C.E.C. et la surface interne pourraient être mesurées par toutes autres méthodes reconnues sur le plan national ou international.

## Revendications

1. Procédé de détermination de l'activité biologique d'un sol, qui comprend l'établissement d'une corrélation entre, d'une part, la teneur en phosphatase alcaline d'une série de sols de référence et, d'autre part, d'autres caractéristiques physiques et/ou chimiques de cette série de sols de référence, lesdites autres caractéristiques étant choisies entre la surface interne du sol, sa teneur en carbone organique et sa capacité d'échange cationique, caractérisé en ce qu'il consiste :
   - à choisir, comme sols de référence, des sols dont l'activité biologique est jugée satisfaisante,
   - à constituer un fichier à partir des informations ainsi recueillies sur lesdits sols de référence,
   - à mesurer la teneur en phosphatase alcaline et l'une desdites autres caractéristiques physico-chimiques précitées du sol objet de la détermination,

- à extraire, dudit fichier, les teneurs en phosphatase alcaline des sols de référence qui correspondent à la valeur mesurée pour ladite autre caractéristique,
- à comparer ladite teneur en phosphatase alcaline du sol objet de la détermination auxdites teneurs en phosphatase alcaline extraites du fichier, et
- à interpréter le résultat de la comparaison à partir d'hyperboles de confiance.

2. Procédé selon la revendication 1, caractérisé en ce que l'interprétation à partir des hyperboles de confiance est faite comme suit :
- valeur dans les hyperboles à 95 % : bon
- valeur dans les hyperboles à 9∅ % : insuffisant ou fort
- valeur hors des hyperboles à 9∅ % : mauvais excessif.

3. Procédé selon la revendication 1 ou 2, caractérisé en ce qu'il consiste à mesurer la teneur en phosphatase alcaline de façon indirecte, par une mesure de son activité enzymatique.

4. Procédé selon l'une quelconque des revendications 1 à 3, caractérisé en ce que la droite de régression obtenue à partir des couples de mesures
. activité enzymatique de la phosphatase alcaline exprimée en µg de p-nitrophénol produit à partir de phosphate de p-nitrophényle /g de sol sec/heure, portée en ordonnée,
. surface interne du sol exprimée en m²/g de sol, portée en abscisse,
fait apparaître un facteur de régression r de l'ordre de ∅,95.

5. Procédé selon l'une quelconque des revendications 1 à 3, caractérisé en ce que la droite de régression obtenue à partir des couples de mesures
. activité enzymatique de la phosphatase alcaline exprimée en µg de p-nitrophénol produit à partir du phosphate de p-nitrophényle /g de sol sec/heure, portée en ordonnée,
. teneur en carbone organique du sol exprimé en pour mille (‰), portée en abscisse,
fait apparaître un facteur de régression r de l'ordre de ∅,75.

6. Procédé selon l'une quelconque des revendications 1 à 3, caractérisé en ce que la droite de régression obtenue à partir des couples de mesures
. activité enzymatique de la phosphatase alcaline exprimée en µg de p-nitrophénol produit à partir de phosphate de p-nitrophényle /g de sol sec/heure, portée en ordonnée,
. capacité d'échange cationique du sol exprimée en milliéquivalents/g de sol, portée en abscisse,
fait apparaître un facteur de régression r de l'ordre de ∅,65.

## Patentansprüche

1. Verfahren zur Bestimmung der biologischen Aktivität eines Bodens, das die Aufstellung einer Korrelation zwischen dem Gehalt einer Reihe von Vergleichsböden an alkalischer Phosphatase einerseits, und anderer physikalischer und/ oder chemischer Eigenschaften dieser Reihe von Vergleichsböden andererseits umfaßt, wobei bei diesen anderen Eigenschaften zwischen der inneren Oberfläche des Bodens, seinem Gehalt an organischem Kohlenstoff und seiner Kationenaustauschkapazität ausgewählt werden kann, **dadurch gekennzeichnet, daß** es darin besteht:
- als Vergleichsböden solche Böden zu wählen, deren biologische Aktivität hinreichend bekannt ist,
- ausgehend von den so über diese Vergleichsböden erhaltenen Informationen eine Kartei einzurichten,
- den Gehalt an alkalischer Phosphatase und eine der anderen vorher erwähnten physikalisch-chemischen Eigenschaften des zu analysierenden Bodens zu messen,
- aus der Kartei die Gehalte an alkalischer Phosphatase für diejenigen Vergleichsböden zu entnehmen, bei denen der Wert der anderen Eigenschaft mit dem gemessenen Wert übereinstimmt,
- dem Gehalt an alkalischer Phosphatase des zu analysierenden Bodens mit den aus der Kartei entnommenen Gehalten an alkalischer Phosphatase zu vergleichen, und
- das Ergebnis des Vergleichs anhand von Vertrauenshyperbeln zu interpretieren.

2. Verfahren nach Anspruch 1, **dadurch gekennzeichnet, daß** die Interpretation anhand von Vertrauenshyperbeln wie folgt vor sich geht:
- Wert zu 95 % in den Hyperbeln : gut
- Wert zu 90 % in den Hyperbeln : ungenügend oder abnorm
- Wert zu 90 % außerhalb der Hyperbeln : extrem schlecht

3. Verfahren nach Anspruch 1 oder 2, **dadurch gekennzeichnet, daß** es darin besteht, den Gehalt an alkalischer Phosphatase auf indirekte Weise zu messen, und zwar durch eine Messung ihrer Enzymaktivität.

4. Verfahren nach einem der Ansprüche 1 bis 3, **dadurch gekennzeichnet, daß** die Regressionsgerade, die mit den Meßpaaren
   - Enzymaktivität der alkalischen Phosphorate angegeben als Menge des aus p-Nitrophenylphosphat erzeugtem p-Nitrophenol in µg / g trockener Boden / Stunde, angetragen auf die Ordinate,
   - innere Oberfläche des Bodens, angegeben in m²/g Boden, angetragen auf die Abszisse,
   erzeugt wird, einen Regressionsfaktor r in der Größenordnung von 0,95 aufweist.

5. Verfahren nach einem der Ansprüche 1 bis 3, **dadurch gekennzeichnet, daß** die Regressionsgerade, die mit den Meßpaaren
   - Enzymaktivität der alkalischen Phosphatase, angegeben als Menge des aus p-Nitrophenylphosphat erzeugtem p-Nitrophenol in µg / g trockener Boden / Stunde, angetragen auf die Ordinate,
   - Gehalt des Bodens an organischem Kohlenstoff in Promille (‰), angetragen auf die Abszisse,
   erzeugt wird, einen Regressionsfaktor r in der Größenordnung von 0,75 aufweist.

6. Verfahren nach einem der Ansprüche 1 bis 3, **dadurch gekennzeichnet, daß** die Regressionsgerade, die mit den Meßpaaren
   - Enzymaktivität der alkalischen Phosphatase, angegeben als Menge des aus p-Nitrophenylphosphat erzeugtem p-Nitrophenol in µg / g trockener Boden / Stunde, angetragen auf die Ordinate,
   - Ionenaustauschkapazität des Bodens in Tausendsteläquivalenten /g Boden, eingetragen auf die Abszisse,
   erzeugt wird, einen Regressionsfaktor r in der Größenordnung von 0,65 aufweist.


**Claims**

1. Method for determining the biological activity of a soil, which comprises the establishment of a correlation between, on the one hand, the alkaline phosphatase content of a series of reference soils and, on the other hand, other physical and/or chemical characteristics of this series of reference soils, said other characteristics being chosen between the interior surface of the soil, its organic carbon content and its capacity of cationic exchange, characterized in that it consists:
   - in choosing, as reference soils, soils whose biological activity is judged satisfactory,
   - in constituting an index from the data thus collected on said reference soils,
   - in measuring the alkaline phosphatase content and one of said other physico-chemical characteristics mentioned above of the soil forming the subject matter of the determination,
   - in extracting from said index the alkaline phosphatase contents of the reference soils which correspond to the value measured for said other characteristic,
   - in comparing said alkaline phosphatase content of the soil forming the subject matter of the determination, with said alkaline phosphatase contents extracted from the index, and
   - in interpreting the result of the comparison from confidence hyperbolas.

2. Method according to Claim 1, characterized in that the interpretation from the confidence hyperbolas is made as follows:
   - value within the hyperbolas at 95%: good
   - value within the hyperbolas at 90%: insufficient or high
   - value outside the hyperbolas at 90%: poor, excessive.

3. Method according to Claim 1 or 2, characterized in that it consists in measuring the alkaline phosphatase content indirectly, by measuring its enzymatic activity.

4. Method according to any one of Claims 1 to 3, characterized in that the straight regression line obtained from the couples of measurements
   . enzymatic activity of the alkaline phosphatase expressed in µg of p-nitrophenol produced from p-nitrophenyl phosphate/g of dry soil/hour, plotted on the y-axis,
   . interior surface of the soil expressed in m²/g of soil, plotted on the x-axis,
   shows a factor of regression r of the order of 0.95.

5. Method according to any one of Claims 1 to 3, characterized in that the straight regression line obtained from the couples of measurements
   . enzymatic activity of the alkaline phosphatase expressed in µg of p-nitrophenol produced from the p-nitrophenyl phosphate/g of dry soil/hour, plotted on the y-axis
   . organic carbon content of the soil expressed in per thousand (°/oo), plotted on the x-axis,
   shows a factor of regression r of the order of 0.75.

6. Method according to any one of Claims 1 to 3, characterized in that the straight regression line obtained from the couples of measurements

. enzymatic activity of the alkaline phosphatase expressed in μg of p-nitrophenol produced from p-nitrophenyl phosphate/g of dry soil/hour, plotted on the y-axis,

. capacity of cationic exchange of the soil expressed in milliequivalents/g of soil, plotted on the x-axis,

shows a factor of regression $r$ of the order of 0.65.

FIG1

ACTIVITE DE LA
PHOSPHATASE
$\mu$g p-NP/g/h

r=0.955

SURFACE INTERNE DU
SOL EN $m^2$/g

EP 0 449 924 B1

FIG2

ACTIVITE DE LA PHOSPHATASE

µg p-NP/g/h

r: 0.7502

TENEUR EN CARBONE DU SOL °/₀₀

EP 0 449 924 B1

FIG 3

r=0.64.82